(19) Europäisches Patentamt / European Patent Office / Office européen des brevets

(11) **EP 3 954 728 A1**

(12) **EUROPEAN PATENT APPLICATION**
published in accordance with Art. 153(4) EPC

(43) Date of publication:
**16.02.2022 Bulletin 2022/07**

(21) Application number: **20795401.7**

(22) Date of filing: **16.04.2020**

(51) International Patent Classification (IPC):
**C08J 3/16** $^{(2006.01)}$     **A61F 13/53** $^{(2006.01)}$
**C08J 3/24** $^{(2006.01)}$     **C08L 101/14** $^{(2006.01)}$

(52) Cooperative Patent Classification (CPC):
**A61F 13/53; C08J 3/16; C08J 3/24; C08L 101/14**

(86) International application number:
**PCT/JP2020/016741**

(87) International publication number:
**WO 2020/218158 (29.10.2020 Gazette 2020/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.04.2019 JP 2019082025**

(71) Applicant: **Sumitomo Seika Chemicals Co., Ltd.
Kako-gun, Hyogo 675-0145 (JP)**

(72) Inventor: **ITO Takashi
Himeji-shi, Hyogo 672-8076 (JP)**

(74) Representative: **Vossius & Partner
Patentanwälte Rechtsanwälte mbB
Siebertstraße 3
81675 München (DE)**

(54) **WATER ABSORBENT RESIN PARTICLES**

(57) Water-absorbent resin particles in which an absorption efficiency index represented by the following formula is 0 to 15 are disclosed. Absorption efficiency index = [(C - A)/A] × 100. A: a physiological saline absorption amount of the water-absorbent resin particles. C: an apparent water absorption amount per 1 g of the water-absorbent resin particles = (B1 - B2)/10. B1: a water absorption amount of an absorbent article for B1 measurement containing the water-absorbent resin particles and including a sheet shaped absorber formed by uniformly mixing 10 g of the water-absorbent resin particles and 8 g of pulverized pulp by air mixing. B2: a water absorption amount of an absorbent article for B2 measurement having the same structure as the absorbent article for B1 measurement except 10 g of the water-absorbent resin particles are not contained.

**Description**

**WATER ABSORBENT RESIN PARTICLES**

**Technical Field**

The present invention relates to water-absorbent resin particles.

**Background Art**

A water-absorbent resin is used in the field of sanitary products, and specifically, it is used as a material for an absorber contained in an absorbent article such as a diaper (for example, Patent Literature 1).

**Citation List**

**Patent Literature**

[Patent Literature 1] Japanese Unexamined Patent Publication No. H5-184622

**Summary of Invention**

**Technical Problem**

When using an absorbent article such as a diaper, for example, in the front of the body of a wearer in a sitting state, a sheet shaped absorber containing water-absorbent resin particles in a state of being inclined along the body comes into contact with urine. Therefore, in the absorbent article such as a diaper, it is required that a liquid flowing on the surface in an inclined state is quickly absorbed, and the speed of sucking up the liquid flowing on the surface and accumulated is high. Furthermore, the above-mentioned performance is required to be maintained even at the time of repeated use.

An object of the present invention is to provide water-absorbent resin particles that provide an absorbent article having better absorption performance in an inclined state.

**Solution to Problem**

Water-absorbent resin particles of the present invention are water-absorbent resin particles in which an absorption efficiency index represented by the following formula is 0 to 15.

$$\text{Absorption efficiency index} = [(C-A)/A] \times 100$$

A: a physiological saline absorption amount of the water-absorbent resin particles
C: an apparent water absorption amount per 1 g of the water-absorbent resin particles = (B1 - B2)/10
B1: a water absorption amount of an absorbent article for B1 measurement containing the water-absorbent resin particles and including a sheet shaped absorber formed by uniformly mixing 10 g of the water-absorbent resin particles and 8 g of pulverized pulp by air mixing
B2: a water absorption amount of an absorbent article for B2 measurement having the same structure as the absorbent article for B1 measurement except 10 g of the water-absorbent resin particles are not contained

In the above-mentioned water-absorbent resin particles, a 5-minute value of non-pressurization DW is preferably 30 ml/g or more.
In the above-mentioned water-absorbent resin particles, a physiological saline absorption amount is preferably 30 g/g or more.
The present invention further provides an absorber containing the above-mentioned water-absorbent resin particles.
The present invention still further provides an absorbent article including the above-mentioned absorber.
The above-mentioned absorbent article may be a diaper.
The present invention still further provides a method for producing water-absorbent resin particles, the method including sorting out water-absorbent resin particles in which an absorption efficiency index represented by the following formula is 0 to 15.

$$\text{Absorption efficiency index} = [(C - A)/A] \times 100$$

A: a physiological saline absorption amount of the water-absorbent resin particles
C: an apparent water absorption amount per 1 g of the water-absorbent resin particles = (B1 - B2)/10
B1: a water absorption amount of an absorbent article for B1 measurement containing the water-absorbent resin particles and including a sheet shaped absorber formed by uniformly mixing 10 g of the water-absorbent resin particles and 8 g of pulverized pulp by air mixing
B2: a water absorption amount of an absorbent article for B2 measurement having the same structure as the absorbent article for B1 measurement except 10 g of the water-absorbent resin particles are not contained

By using the water-absorbent resin particles obtained by the above-mentioned production method, it is possible to obtain an absorbent article having better absorption performance in an inclined state.

The present invention still further provides a method for improving absorption performance of an absorbent article containing water-absorbent resin particles in an inclined state, the method including adjusting an absorption efficiency index of the water-absorbent resin particles represented by the following formula to 0 to 15.

$$\text{Absorption efficiency index} = [(C - A)/A] \times 100$$

A: a physiological saline absorption amount of the water-absorbent resin particles
C: an apparent water absorption amount per 1 g of the water-absorbent resin particles = (B1 - B2)/10
B1: a water absorption amount of an absorbent article for B1 measurement containing the water-absorbent resin particles and including a sheet shaped absorber formed by uniformly mixing 10 g of the water-absorbent resin particles and 8 g of pulverized pulp by air mixing
B2: a water absorption amount of an absorbent article for B2 measurement having the same structure as the absorbent article for B1 measurement except 10 g of the water-absorbent resin particles are not contained

**Advantageous Effects of Invention**

According to the present invention, water-absorbent resin particles that provide an absorbent article having better absorption performance in an inclined state are provided.

**Brief Description of Drawings**

Fig. 1 is a cross-sectional view showing an example of an absorbent article.
Fig. 2 is a plan view showing an outline of a stirring blade (flat plate blade having slits on a flat plate portion).
Fig. 3 is a schematic view showing an absorbent article for measuring a water absorption amount.
Fig. 4 is a schematic view showing a method for measuring non-pressurization DW.
Fig. 5 is a schematic view showing a method for measuring a water absorption amount under load.
Fig. 6 is a schematic view showing a method for measuring a liquid accumulation absorption time.

**Description of Embodiments**

Hereinafter, embodiments of the present invention will be described in detail. However, the present invention is not limited to the following embodiments, and can be various modified and implemented within the scope of the gist thereof.

In the present specification, "acrylic" and "methacrylic" are collectively referred to as "(meth)acrylic". "Acrylate" and "methacrylate" are also referred to as "(meth)acrylate". "(Poly)" means both of a case where there is a prefix of "poly" and a case where there is no prefix thereof. In a numerical value range described in a stepwise manner in the present specification, an upper limit value or a lower limit value of a numerical value range in a certain step can be arbitrarily combined with an upper limit value or a lower limit value of a numerical value range in another step. In a numerical value range described in the present specification, an upper limit value or a lower limit value of the numerical value range may be replaced with a value shown in Examples. "Water-soluble" means that a solubility of 5% by mass or more is exhibited in water at 25°C. For materials exemplified in the present specification, one kind may be used alone, or two or more kinds may be used in combination. In a case where there are a plurality of substances corresponding to each of components in a composition, a content of each of the components in the composition means the total amount of the plurality of substances present in the composition unless otherwise specified. "Physiological saline" refers to an aqueous solution of 0.9% by mass sodium chloride.

Water-absorbent resin particles of the present embodiment are water-absorbent resin particles in which an absorption efficiency index represented by the following formula is in the range of 0 to 15.

$$\text{Absorption efficiency index} = [(C - A)/A] \times 100$$

A: a physiological saline absorption amount of the water-absorbent resin particles
C: an apparent water absorption amount per 1 g of the water-absorbent resin particles = (B1 - B2)/10
B1: a water absorption amount of an absorbent article for B1 measurement containing the water-absorbent resin particles and including a sheet shaped absorber formed by uniformly mixing 10 g of the water-absorbent resin particles and 8 g of pulverized pulp by air mixing
B2: a water absorption amount of an absorbent article for B2 measurement having the same structure as the absorbent article for B1 measurement except 10 g of the water-absorbent resin particles are not contained

The physiological saline absorption amount (A) of the water-absorbent resin particles indicates the maximum water-absorbing ability of the water-absorbent resin particles alone. Meanwhile, in an absorbent article, the water-absorbent resin particles are generally used for an absorber together with a fibrous substance such as pulp, but particularly in the presence of the fibrous substance, the water-absorbent resin particles may not fully exhibit their original water-absorbing ability. In the calculation formula of the absorption efficiency index, the apparent water absorption amount (C) per 1 g of the water-absorbent resin particles indicates the contribution part to the water absorption amount of the water-absorbent resin particles alone when used together with the fibrous substance as the absorbent article. The absorption efficiency index of the present embodiment can be said to be an index showing how much the water-absorbing ability originally possessed by the water-absorbent resin particles or a higher water-absorbing ability can be exhibited when the water-absorbent resin particles are used for the absorbent article.

When the water-absorbent resin particles in which the absorption efficiency index is 0 to 15 are used for the absorbent article, absorption performance of the absorbent article in an inclined state is improved. The reason why such an effect is obtained is not clear, but the inventors of the present invention speculate as follows. However, the present invention is not limited to the following mechanism. In the absorbent article, in an absorber containing the water-absorbent resin particles and a fibrous substance, excess water, that is, water absorbed in the absorber but not sufficiently retained may be generated. It is thought that, in the water-absorbent resin particles of the present embodiment in which the absorption efficiency index is in a predetermined range, because crosslinking in the particles is more uniform, more uniform swelling in the particles is possible when absorbing water, excess water in the absorber can be more efficiently absorbed, and water can be absorbed with an efficiency equal to or higher than the water absorption amount of the water-absorbent resin particles alone.

In the water-absorbent resin particles of the present embodiment, the absorption efficiency index may be 1 or more, 2 or more, 3 or more, 4 or more, 5 or more, 6 or more, or 7 or more, and may be 15 or less, 14 or less, or 13 or less. The absorption efficiency index may be 1 to 15, 3 to 14, 5 to 14, or 7 to 13.

For example, the physiological saline absorption amount (A) of the water-absorbent resin particles of the present embodiment may be 30 g/g or more, 35 g/g or more, 40 g/g or more, 45 g/g or more, 50 g/g or more, 55 g/g or more, or 60 g/g or more, and may be 90 g/g or less, 85 g/g or less, 80 g/g or less, 75 g/g or less, 70 g/g or less, or 65 g/g or less. From the viewpoint of easily enhancing absorption performance of the absorbent article, the physiological saline absorption amount (A) of the water-absorbent resin particles of the present embodiment is preferably 30 to 90 g/g, more preferably 40 to 80 g/g, further more preferably 50 to 75 g/g, still further more preferably 55 to 70 g/g, and particularly preferably 60 to 65 g/g. A method for measuring the physiological saline absorption amount will be described in detail in Examples to be described later.

In the water-absorbent resin particles of the present embodiment, for example, the apparent water absorption amount (C) per 1 g of the water-absorbent resin particles may be 50 to 80 g, is preferably 60 to 75 g, more preferably 55 to 75 g/g, and further more preferably 60 to 72 g/g, from the viewpoint of exhibiting higher absorption performance in the absorbent article. A specific method for measuring the water absorption amount (B1) of the absorbent article for B1 measurement containing the water-absorbent resin particles and the water absorption amount (B2) of the absorbent article for B2 measurement not containing the water-absorbent resin particles, which are used to calculate the apparent water absorption amount (C) per 1 g of the water-absorbent resin particles, will be described in Examples to be described later.

In the water-absorbent resin particles of the present embodiment, a 5-minute value of non-pressurization DW may be 30 to 80 ml/g. The 5-minute value of non-pressurization DW is preferably 37 ml/g or more, more preferably 42 ml/g or more, and further more preferably 45 ml/g or more from the viewpoint of easily increasing the water absorption amount of the absorbent article. The 5-minute value of non-pressurization DW may be 70 ml/g or less, for example.

The water-absorbent resin particles of the present embodiment exhibit high absorption performance in the absorbent article in an inclined state at a portion almost not bearing the body weight, such as the front of the body of an absorbent article wearer, but also has better absorption performance under load. In the water-absorbent resin particles of the present embodiment, for example, the water absorption amount under the load of 4.14 kPa may be 10 ml/g or more, 15 ml/g or more, 18 ml/g or more, or 20 ml/g or more, and may be 35 ml/g or less, 30 ml/g or less, or 25 ml/g or less. In the water-absorbent resin particles of the present embodiment, for example, the water absorption amount under the load of 2.07 kPa may be 15 mlg/l or more, 20 ml/g or more, 25 ml/g or more, or 30 ml/g or more, and may be 45 ml/g or less or 40 ml/g or less.

Examples of the shape of the water-absorbent resin particles of the present embodiment include a substantially spherical shape, a crushed shape, and a granular shape. Furthermore, the water-absorbent resin particles of the present embodiment may be in the form (secondary particles) in which fine particles (primary particles) are aggregated, in addition to the form in which each is composed of a single particle. The median particle diameter of the water-absorbent resin particles (water-absorbent resin particles before water absorption) of the present embodiment may be 250 to 850 $\mu$m, 300 to 700 $\mu$m, or 300 to 600 $\mu$m. The water-absorbent resin particles of the present embodiment may have a desired particle size distribution at the time of being obtained by a production method to be described later, but the particle size distribution may be adjusted by performing an operation such as particle size adjustment using classification with a sieve.

The water-absorbent resin particles of the present embodiment can contain a crosslinked polymer (a crosslinked polymer having a structural unit derived from an ethylenically unsaturated monomer) obtained by polymerizing a monomer containing an ethylenically unsaturated monomer, as polymer particles, for example. That is, the water-absorbent resin particles of the present embodiment can have a polymer having structural unit derived from an ethylenically unsaturated monomer, and can contain polymer particles including a crosslinked polymer having a structural unit derived from an ethylenically unsaturated monomer. As the ethylenically unsaturated monomer, a water-soluble ethylenically unsaturated monomer can be used. Examples of the polymerization method include a reverse phase suspension polymerization method, an aqueous solution polymerization method, a bulk polymerization method, and a precipitation polymerization method. Among these, the reverse phase suspension polymerization method or the aqueous solution polymerization method is preferable from the viewpoint of ensuring good water-absorbent characteristics of the obtained water-absorbent resin particles and facilitating control of the polymerization reaction. In the following, as a method for polymerizing an ethylenically unsaturated monomer, a reverse phase suspension polymerization method will be described as an example.

The ethylenically unsaturated monomer is preferably water-soluble, and examples thereof include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where the ethylenically unsaturated monomer has an amino group, the amino group may be quaternized. The ethylenically unsaturated monomer may be used alone, or may be used in combination of two or more kinds thereof. The functional group, such as a carboxyl group and an amino group, of the above-mentioned monomer can function as a functional group capable of crosslinking in a surface crosslinking step to be described later.

Among these, from the viewpoint of industrial availability, the ethylenically unsaturated monomer preferably contains at least one compound selected from the group consisting of (meth)acrylic acid and a salt thereof, acrylamide, methacrylamide, and N,N-dimethyl acrylamide, and more preferably contains at least one compound selected from the group consisting of (meth)acrylic acid and a salt thereof, and acrylamide. From the viewpoint of further enhancing water-absorbent characteristics, the ethylenically unsaturated monomer further more preferably contains at least one compound selected from the group consisting of (meth)acrylic acid and a salt thereof. That is, the water-absorbent resin particles preferably have a structural unit derived from at least one selected from the group consisting of (meth)acrylic acid and a salt thereof.

As the monomer for obtaining the water-absorbent resin particles, a monomer other than the above-mentioned ethylenically unsaturated monomer may be used. Such a monomer can be used by being mixed with an aqueous solution containing the above-mentioned ethylenically unsaturated monomer, for example. The use amount of the ethylenically unsaturated monomer may be 70 to 100 mol%, and may be 80 to 100 mol%, 90 to 100 mol%, 95 to 100 mol%, or 100 mol% with respect to the total amount of the monomer (the total amount of the monomer for obtaining the water-absorbent resin particles. For example, the total amount of the monomers that provide a structural unit of the crosslinked polymer. The same applies hereinafter). Among these, the ratio of (meth)acrylic acid and a salt thereof may be 70 to 100 mol%, and may be 80 to 100 mol%, 90 to 100 mol%, 95 to 100 mol%, or 100 mol% with respect to the total amount of the monomers. "Ratio of (meth)acrylic acid and a salt thereof" means the ratio of the total amount of (meth)acrylic acid and a salt thereof.

According to the present embodiment, as an example of the water-absorbent resin particles, it is possible to provide the water-absorbent resin particle containing a crosslinked polymer having a structural unit derived from an ethylenically unsaturated monomer, in which the ethylenically unsaturated monomer contains at least one compound selected from the group consisting of (meth)acrylic acid and a salt thereof, and the ratio of (meth)acrylic acid and a salt thereof is 70 to 100 mol% with respect to the total amount of the monomer for obtaining the water-absorbent resin particles.

The ethylenically unsaturated monomer is usually preferably used as an aqueous solution. The concentration of the ethylenically unsaturated monomer in the aqueous solution containing the ethylenically unsaturated monomer (hereinafter, simply referred to as "monomer aqueous solution") is preferably 20% by mass or more and a saturated concentration or less, more preferably 25 to 70% by mass, and further more preferably 30 to 55% by mass. Examples of the water used in the aqueous solution include tap water, distilled water, and ion-exchanged water.

In a case where the ethylenically unsaturated monomer has an acid group, the monomer aqueous solution may be used by neutralizing the acid group with an alkaline neutralizing agent. The degree of neutralization of the ethylenically unsaturated monomer by the alkaline neutralizing agent is preferably 10 to 100 mol%, more preferably 50 to 90 mol%, and further more preferably 60 to 80 mol% of the acid group in the ethylenically unsaturated monomer, from the viewpoint of increasing an osmotic pressure of the obtained water-absorbent resin particles, and further enhancing water-absorbent characteristics. Examples of the alkaline neutralizing agent include alkali metal salts such as sodium hydroxide, sodium carbonate, sodium hydrogen carbonate, potassium hydroxide, and potassium carbonate; and ammonia. The alkaline neutralizing agent may be used alone, or may be used in combination of two or more kinds thereof. The alkaline neutralizing agent may be used in the form of an aqueous solution to simplify the neutralization operation. Neutralization of the acid group of the ethylenically unsaturated monomer can be performed by adding an aqueous solution of sodium hydroxide, potassium hydroxide, or the like dropwise in the above-mentioned monomer aqueous solution and mixing therewith.

In a reverse phase suspension polymerization method, a monomer aqueous solution is dispersed in a hydrocarbon dispersion medium in the presence of a surfactant, and polymerization of the ethylenically unsaturated monomer can be performed using a radical polymerization initiator or the like.

Examples of the surfactant include a nonionic surfactant and an anionic surfactant. Examples of the nonionic surfactant include sorbitan fatty acid esters, polyglycerin fatty acid esters, sucrose fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene hydrogenated castor oil, alkylallyl formaldehyde condensed polyoxyethylene ethers, polyoxyethylene polyoxypropylene block copolymers, polyoxyethylene polyoxypropyl alkyl ethers, and polyethylene glycol fatty acid esters. Examples of the anionic surfactant include fatty acid salts, alkylbenzene sulfonate, alkylmethyl taurate, polyoxyethylene alkylphenyl ether sulfuric acid ester salts, polyoxyethylene alkyl ether sulfonic acid salts, phosphoric acid esters of polyoxyethylene alkyl ethers, and phosphoric acid esters of polyoxyethylene alkyl allyl ethers. The surfactant may be used alone, or may be used in combination of two or more kinds thereof.

From the viewpoint of a good state of the W/O type reverse phase suspension, easily obtaining water-absorbent resin particles having a suitable particle diameter, and industrial availability, the surfactant preferably contains at least one compound selected from the group consisting of sorbitan fatty acid esters, polyglycerin fatty acid esters, and sucrose fatty acid esters. From the viewpoint of easily obtaining an appropriate particle size distribution of the water-absorbent resin particles, and from the viewpoint of easily improving water-absorbent characteristics of the water-absorbent resin particles and performances of the absorber and the absorbent article using the same, the surfactant preferably contains sucrose fatty acid ester, and more preferably contains sucrose stearic acid ester.

The use amount of the surfactant is preferably 0.05 to 10 parts by mass, more preferably 0.08 to 5 parts by mass, and further more preferably 0.1 to 3 parts by mass with respect to 100 parts by mass of the monomer aqueous solution, from the viewpoint of obtaining a sufficient effect on the use amount and economic efficiency.

In the reverse phase suspension polymerization, a polymeric dispersant may be used in combination with the above-mentioned surfactant. Examples of the polymeric dispersant include maleic anhydride-modified polyethylene, maleic anhydride-modified polypropylene, a maleic anhydride-modified ethylene-propylene copolymer, a maleic anhydride-modified EPDM (ethylene propylene diene terpolymer), maleic anhydride-modified polybutadiene, a maleic anhydride-ethylene copolymer, a maleic anhydride-propylene copolymer, a maleic anhydride-ethylene-propylene copolymer, a maleic anhydride-butadiene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, and ethyl hydroxyethyl cellulose. The polymeric dispersant may be used alone or may be used in combination of two or more kinds thereof. From the viewpoint of better dispersion stability of the monomer, the polymeric dispersant is preferably at least one selected from the group consisting of maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene, and an oxidized ethylene-propylene copolymer.

The use amount of the polymeric dispersant is preferably 0.05 to 10 parts by mass, more preferably 0.08 to 5 parts by mass, and further more preferably 0.1 to 3 parts by mass with respect to 100 parts by mass of the monomer aqueous solution, from the viewpoint of obtaining a sufficient effect on the use amount and economic efficiency.

The hydrocarbon dispersion medium may contain at least one compound selected from the group consisting of chain aliphatic hydrocarbons having 6 to 8 carbon atoms and alicyclic hydrocarbons having 6 to 8 carbon atoms. Examples of the hydrocarbon dispersion medium include chain aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. The hydrocarbon dispersion medium may be used alone, or may be used in combination of two or more kinds thereof.

The hydrocarbon dispersion medium may contain at least one selected from the group consisting of n-heptane and cyclohexane from the viewpoint of industrial availability and stable quality. In addition, from the same viewpoint, as the mixture of the above-mentioned hydrocarbon dispersion medium, for example, a commercially available Exxsol Heptane (manufactured by ExxonMobil: containing 75% to 85% of n-heptane and isomeric hydrocarbons) may be used.

The use amount of the hydrocarbon dispersion medium is preferably 30 to 1000 parts by mass, more preferably 40 to 500 parts by mass, and further more preferably 50 to 400 parts by mass with respect to 100 parts by mass of the monomer aqueous solution, from the viewpoint of appropriately removing the heat of polymerization and easily controlling the polymerization temperature. In a case where the use amount of the hydrocarbon dispersion medium is 30 parts by mass or more, the polymerization temperature tends to be easily controlled. In a case where the use amount of the hydrocarbon dispersion medium is 1000 parts by mass or less, the productivity of polymerization tends to be improved, which is economical.

The radical polymerization initiator is preferably water-soluble, and examples thereof include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis[2-(2-imidazolin-2-yl)propane] dihydrochloride, 2,2'-azobis{2-[1-(2-hydroxyethyl)-2-imidazolin-2-yl]propane} dihydrochloride, 2,2' -azobis {2-methyl-N-[1,1-bis(hydroxymethyl)-2-hydroxyethyl]propi onamide}, 2,2'-azobis[2-methyl-N-(2-hydroxyethyl)-propionamide], and 4,4'-azobis(4-cyanovaleric acid). The radical polymerization initiator may be used alone, or may be used in combination of two or more kinds thereof. The radical polymerization initiator is preferably at least one selected from the group consisting of potassium persulfate, ammonium persulfate, sodium persulfate, 2,2'-azobis(2-amidinopropane) dihydrochloride, 2,2'-azobis[2-(2-imidazolin-2-yl)propane] dihydrochloride, and 2,2'-azobis{2-[1-(2-hydroxyethyl)-2-imidazolin-2-yl]propane} dihydrochloride; more preferably potassium persulfate, ammonium persulfate, sodium persulfate; and further more preferably sodium persulfate.

The use amount of the radical polymerization initiator may be 0.05 to 10 mmol with respect to 1 mol of the ethylenically unsaturated monomer. In a case where the use amount of the radical polymerization initiator is 0.05 mmol or more, the polymerization reaction does not require a long time and is efficient. In a case where the use amount of the radical polymerization initiator is 10 mmol or less, the occurrence of a rapid polymerization reaction is easily inhibited.

The above-mentioned radical polymerization initiator can also be used as a redox polymerization initiator in combination with a reducing agent such as sodium sulfite, sodium hydrogen sulfite, ferrous sulfate, and L-ascorbic acid.

At the time of the polymerization reaction, the monomer aqueous solution used for the polymerization may contain a chain transfer agent. Examples of the chain transfer agent include hypophosphites, thiols, thiolic acids, secondary alcohols, and amines.

The monomer aqueous solution used for the polymerization may contain a thickener in order to control the particle diameter of the water-absorbent resin particles. Examples of the thickener include hydroxyethyl cellulose, hydroxypropyl cellulose, methyl cellulose, carboxymethyl cellulose, polyethylene glycol, polyacrylamide, polyethyleneimine, dextrin, sodium alginate, polyvinyl alcohol, polyvinylpyrrolidone, and polyethylene oxide. In a case where the stirring speed at the time of polymerization is the same, the higher the viscosity of the monomer aqueous solution, the larger the median particle diameter of the obtained particles tends to be.

Internal crosslinking by self-crosslinking may occur during polymerization, but crosslinking may be performed by using an internal crosslinking agent. In a case where an internal crosslinking agent is used, water-absorbent characteristics of the water-absorbent resin particles are easily controlled. The internal crosslinking agent is usually added to a reaction solution during the polymerization reaction. Examples of the internal crosslinking agent include di or tri (meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the above-mentioned polyols with unsaturated acids (such as maleic acid and fumaric acid); bis(meth)acrylamides such as N,N'-methylenebis(meth)acrylamide; di or tri (meth)acrylic acid esters obtained by reacting a polyepoxide with (meth)acrylic acid; carbamyl di(meth)acrylate esters obtained by reacting a polyisocyanate (such as tolylene diisocyanate and hexamethylene diisocyanate) with hydroxyethyl (meth)acrylate; compounds having two or more polymerizable unsaturated groups, such as allylated starch, allylated cellulose, diallyl phthalate, N,N',N''-triallyl isocyanurate, and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromohydrin, and $\alpha$-methyl epichlorohydrin; and compounds having two or more reactive functional groups, such as isocyanate compounds (such as 2,4-tolylene diisocyanate and hexamethylene diisocyanate). The internal crosslinking agent may be used alone, or may be used in combination of two or more kinds thereof. The internal crosslinking agent is preferably a polyglycidyl compound, is more preferably a diglycidyl ether compound, and is further more preferably at least one selected from the group consisting of (poly)ethylene glycol diglycidyl ether, (poly)propylene glycol diglycidyl ether, and (poly)glycerin diglycidyl ether.

The use amount of the internal crosslinking agent is preferably 30 mmol or less, more preferably 0.01 to 10 mmol, further more preferably 0.012 to 5 mmol, particularly preferably 0.015 to 1 mmol, extremely preferably 0.02 to 0.1 mmol, and extraordinarily preferably 0.025 to 0.08 mmol, per 1 mol of the ethylenically unsaturated monomer, from the viewpoint of easily obtaining better absorption performance in the absorbent article, and from the viewpoint of suppressing water-soluble property by appropriately crosslinking the obtained polymer to easily obtain the sufficient water absorption amount.

It is possible to perform heating while stirring in a state of mixing an ethylenically unsaturated monomer, a radical polymerization initiator, an aqueous phase containing an internal crosslinking agent if necessary, a hydrocarbon dispersion medium, a surfactant, and an oil phase containing a polymeric dispersant or the like, and to perform reverse phase suspension polymerization in a water-in-oil system.

When performing the reverse phase suspension polymerization, a monomer aqueous solution containing an ethylenically unsaturated monomer is dispersed in a hydrocarbon dispersion medium in the presence of a surfactant (if necessary, additionally a polymeric dispersant). At this time, before the start of the polymerization reaction, the timing of adding the surfactant, the polymeric dispersant, or the like may be either before or after the addition of the monomer aqueous solution.

Among these, from the viewpoint of easily reducing the amount of the hydrocarbon dispersion medium remaining in the obtained water-absorbent resin, it is preferable to perform polymerization after dispersing the monomer aqueous solution in the hydrocarbon dispersion medium in which the polymeric dispersant is dispersed and then further dispersing the surfactant.

Reverse phase suspension polymerization can be performed in one stage, or in multiple stages of two or more stages. Reverse phase suspension polymerization is preferably performed in two to three stages from the viewpoint of increasing productivity.

In a case where reverse phase suspension polymerization is performed in multiple stages of two or more stages, a first stage reverse phase suspension polymerization is performed, an ethylenically unsaturated monomer is added to the reaction mixture obtained in the first polymerization reaction and mixed therewith, and second and subsequent stages of reverse phase suspension polymerization may be performed in the same method as the first stage. In the reverse phase suspension polymerization in each stage of the second and subsequent stages, in addition to the ethylenically unsaturated monomer, the above-mentioned radical polymerization initiator and/or internal crosslinking agent is preferably added in a range of a molar ratio of each component with respect to the above-mentioned ethylenically unsaturated monomer, based on an amount of the ethylenically unsaturated monomer added at the time of the second and subsequent stages of reverse phase suspension polymerization, to perform reverse phase suspension polymerization. In the reverse phase suspension polymerization in each stage of second and subsequent stages, an internal crosslinking agent may be used if necessary. In a case of using the internal crosslinking agent, the internal crosslinking agent is preferably added within a range of the molar ratio of each component with respect to the above-mentioned ethylenically unsaturated monomer based on the amount of the ethylenically unsaturated monomer provided in each stage, to perform reverse phase suspension polymerization.

The temperature of the polymerization reaction varies depending on the used radical polymerization initiator, and the temperature is preferably 20°C to 150°C, and more preferably 40°C to 120°C, from the viewpoint of rapidly proceeding the polymerization and shortening the polymerization time to enhance economic efficiency, and easily removing polymerization heat and smoothly performing reaction. The reaction time is usually 0.5 to 4 hours. The completion of the polymerization reaction can be confirmed by stopping the temperature rise in the reaction system. Thus, the polymer of the ethylenically unsaturated monomer is usually obtained in a state of a hydrogel.

After the polymerization, a post-polymerization crosslinking agent may be added to the obtained hydrogel-like polymer and heated to perform crosslinking. By performing post-polymerization crosslinking, a degree of crosslinking of the hydrogel-like polymer can be increased, and water-absorbent characteristics can be further improved.

Examples of the crosslinking agent for performing post-polymerization include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups, such as (poly)ethylene glycol diglycidyl ether, (poly)propylene glycol diglycidyl ether, and (poly)glycerin diglycidyl ether; haloepoxy compounds such as epichlorohydrin, epibromohydrin, and α-methyl epichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis[N,N-di(β-hydroxyethyl)]adipamide. Among these, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. The crosslinking agent may be used alone, or may be used in combination of two or more kinds thereof.

The amount of the post-polymerization crosslinking agent may be 30 mmol or less, 10 mmol or less, or 0.01 to 5 mmol per 1 mol of the ethylenically unsaturated monomer, from the viewpoint of easily obtaining suitable water-absorbent characteristics.

The timing of adding the post-polymerization crosslinking agent may be after the polymerization of the ethylenically unsaturated monomer used for the polymerization, and in the case of multiple-stage polymerization, it is preferable to add after the multiple-stage polymerization. Considering fluctuation in water due to heat generation at the time of polymerization and after polymerization, retention due to process delay, opening of the system at the time of addition of the crosslinking agent, addition of water due to the addition of the crosslinking agent, or the like, the post-polymerization crosslinking agent is preferably added in a region of [water content (immediately after polymerization) ± 3% by mass] from the viewpoint of water content (to be described later).

Subsequently, the polymer particles (for example, polymer particles having a structural unit derived from an ethylenically unsaturated monomer) are obtained by drying in order to remove water from the obtained hydrogel-like polymer. Examples of a drying method include (a) a method of removing water by performing azeotropic distillation by heating from outside in a state where a hydrogel-like polymer is dispersed in a hydrocarbon dispersion medium, and refluxing the hydrocarbon dispersion medium, (b) a method of taking out a hydrogel-like polymer by decantation and drying under reduced pressure, and (c) a method of filtering the hydrogel-like polymer with a filter and drying under reduced pressure. Among these, it is preferable to use the method (a) due to the simplicity in the production process.

It is possible to adjust the particle diameter of water-absorbent resin particles by adjusting a rotation speed of a stirrer during the polymerization reaction, or by adding a flocculant into the system after the polymerization reaction or in the initial stage of drying. By adding a flocculant, it is possible to increase the particle diameter of the obtained water-absorbent resin particles. As the flocculant, an inorganic flocculant can be used. Examples of the inorganic flocculant (for example, powdered inorganic flocculant) include silica, zeolite, bentonite, aluminum oxide, talc, titanium dioxide, kaolin, clay, and hydrotalcite. From the viewpoint of better flocculation effect, the flocculant is preferably at least one selected from the group consisting of silica, aluminum oxide, talc, and kaolin.

In the reverse phase suspension polymerization, a method of adding the flocculant is preferably a method of preliminarily dispersing a flocculant in a hydrocarbon dispersion medium or water of the same type as that used in the polymerization, and then mixing into a hydrocarbon dispersion medium containing a hydrogel-like polymer under stirring.

The addition amount of the flocculant is preferably 0.001 to 1 part by mass, more preferably 0.005 to 0.5 part by mass, and further more preferably 0.01 to 0.2 parts by mass with respect to 100 parts by mass of the ethylenically unsaturated monomer used for the polymerization. In a case where the addition amount of the flocculant is within the above-mentioned range, water-absorbent resin particles having a target particle size distribution can be easily obtained.

In the production of the water-absorbent resin particles, it is preferable to perform surface crosslinking of a surface portion (surface and in the vicinity of surface) of a hydrogel-like polymer using a crosslinking agent in a drying step (water removing step) or any subsequent steps. By performing surface crosslinking, the absorption efficiency index, water-absorbent characteristics, and the like of the water-absorbent resin particles are easily controlled. The surface crosslinking is preferably performed at the timing when the hydrogel-like polymer has a specific water content. The timing of surface crosslinking is preferably when the water content of the hydrogel-like polymer is 5% to 50% by mass, more preferably when the water content of the hydrogel-like polymer is 10% to 40% by mass, and further more preferably when the water content of the hydrogel-like polymer is 15% to 35% by mass.

The water content (mass%) of the hydrogel-like polymer is calculated by the following formula.

$$\text{Water content} = [\text{Ww}/(\text{Ww} + \text{Ws})] \times 100$$

Ww: Water amount of a hydrogel-like polymer obtained by adding water amount used if necessary when mixing a flocculant, a surface crosslinking agent, or the like to an amount obtained by subtracting water amount discharged to the outside of the system in the drying step, from water amount contained in a monomer aqueous solution before polymerization in the entire polymerization step.

Ws: Solid content calculated from the charged amount of materials such as ethylenically unsaturated monomer, crosslinking agent, and initiator that constitute a hydrogel-like polymer.

Examples of the crosslinking agent (surface crosslinking agent) for performing surface crosslinking include compounds having two or more reactive functional groups. Examples of the surface crosslinking agent include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and α-methyl epichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; and hydroxyalkylamide compounds such as bis[N,N-di(β-hydroxyethyl)]adipamide. The surface crosslinking agent may be used alone, or may be used in combination of two or more kinds thereof. The surface crosslinking agent is preferably a polyglycidyl compound, and more preferably at least one selected from the group consisting of (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether.

The use amount of the surface crosslinking agent is preferably 0.01 to 20 mmol, more preferably 0.05 to 10 mmol, further more preferably 0.1 to 5 mmol, particularly preferably 0.12 to 1 mmol, and extremely preferably 0.15 to 0.5 mmol per 1 mol of the ethylenically unsaturated monomer used for polymerization, from the viewpoint of easily obtaining suitable water-absorbent characteristics.

After the surface crosslinking, it is possible to obtain polymer particles which are surface-crosslinked dried products by distilling off water and a hydrocarbon dispersion medium, drying under heating and reduced pressure, or the like with a known method.

The polymerization reaction can be carried out using various stirrers having a stirring blade. As the stirring blade, it is possible to use a flat plate blade, a lattice blade, a paddle blade, a propeller blade, an anchor blade, a turbine blade, a Pfaudler blade, a ribbon blade, a full zone blade, a max blend blade, or the like. A flat plate blade has a shaft (stirring shaft) and a flat plate portion (stirring portion) disposed around the shaft. The flat plate portion may have a slit or the like. In a case where the flat plate blade is used as the stirring blade, it is easy to uniformly carry out the crosslinking reaction in polymer particles, and it is easy to adjust the absorption efficiency index within a desired range while maintaining water-absorbent characteristics.

The water-absorbent resin particles of the present embodiment may be constituted of only the polymer particles, but can further contain additional components such as a gel stabilizer, a metal chelating agent (ethylenediaminetetraacetic acid and a salt thereof, diethylenetriamine pentaacetic acid and a salt thereof, and the like, for example, diethylenetriamine pentaacetic acid pentasodium), and a flowability improver (lubricant) of the polymer particles. Additional components can be disposed inside the polymer particles, on the surface of the polymer particles, or both thereof.

The water-absorbent resin particles may contain a plurality of inorganic particles disposed on the surface of the polymer particles. For example, by mixing the polymer particles and the inorganic particles, it is possible to dispose the inorganic particles on the surface of the polymer particles. The inorganic particles may be silica particles such as amorphous silica.

In a case where the water-absorbent resin particles include inorganic particles disposed on the surface of the polymer particles, the content of the inorganic particles may be within the following range based on the total mass of the polymer particles. The content of the inorganic particles may be 0.05% by mass or more, 0.1% by mass or more, 0.15% by mass or more, or 0.2% by mass or more. The content of the inorganic particles may be 5.0% by mass or less, 3.0% by mass or less, 1.0% by mass or less, 0.5% by mass or less, or 0.3% by mass or less.

The inorganic particles here usually have a minute size as compared with the size of the polymer particles. For example, the average particle diameter of the inorganic particles may be 0.1 to 50 $\mu$m, 0.5 to 30 $\mu$m, or 1 to 20 $\mu$m. The average particle diameter can be measured by a pore electric resistance method or a laser diffraction/scattering method depending on the characteristics of the particles.

The water-absorbent resin particles of the present embodiment have better absorbency for a body fluid such as urine and blood, and can be applied to the field of sanitary products such as paper diapers, sanitary napkins, and tampons, pet sheets, and treatment materials for animal excrement such as dog or cat toilet litters, for example.

The water-absorbent resin particles of the present embodiment can be suitably used for the absorber. The absorber of the present embodiment contains the water-absorbent resin particles of the present embodiment. The content of the water-absorbent resin particles in the absorber is preferably 100 to 1000 g (that is, 100 to 1000 g/m$^2$), more preferably 150 to 800 g/m$^2$, and further more preferably 200 to 700 g/m$^2$, per square meter of the absorber from the viewpoint of obtaining sufficient liquid absorption performances when the absorber is used in the absorbent article. The above-mentioned content is preferably 100 g/m$^2$ or more from the viewpoint of exhibiting sufficient liquid absorption performances as the absorbent article. The above-mentioned content is preferably 1000 g/m$^2$ or less from the viewpoint of inhibiting occurrence of a gel blocking phenomenon.

The absorber may further contain a fibrous substance for example, in addition to the water-absorbent resin particles. The absorber may be a mixture containing the water-absorbent resin particles and the fibrous substance, for example. The mass ratio of the water-absorbent resin particles in the absorber may be 2% to 100% by mass, is preferably 10% to 80% by mass, and more preferably 20% to 70% by mass with respect to a total of the water-absorbent resin particles and the fibrous substance. For example, the structure of the absorber may be a form in which the water-absorbent resin particles and the fibrous substance are uniformly mixed, may be a form in which the water-absorbent resin particles are sandwiched between the fibrous substances formed in the shape of a sheet or a layer, or may be other forms.

Examples of the fibrous substance include finely pulverized wood pulp; cotton; cotton linter; rayon; cellulosic fibers such as cellulose acetate; and synthetic fibers such as polyamide, polyester, and polyolefin. The average fiber length of the fibrous substance is usually 0.1 to 10 mm, and may be 5 to 5 mm. In addition, the fibrous substance may be a mixture of the above-mentioned fibers. From the viewpoint of increasing the water absorption amount of the absorber, finely pulverized wood pulp is more preferable as the fibrous substance.

In order to enhance the morphological retention before and during use of the absorber, the fibers may be adhered to each other by adding an adhesive binder to the fibrous substance. Examples of the adhesive binder include thermal bonding synthetic fibers, hot melt adhesives, and adhesive emulsions.

Examples of the thermal bonding synthetic fiber include a total fusion type binder such as polyethylene, polypropylene, and an ethylene-propylene copolymer; and a non-total fusion type binder made of a side-by-side or core-sheath structure of polypropylene and polyethylene. In the above-mentioned non-total fusion type binder, only the polyethylene portion is thermal-bonded. Examples of the hot melt adhesive include a blend of a base polymer such as ethylene-vinyl acetate copolymer, styrene-isoprene-styrene block copolymer, styrene-butadiene-styrene block copolymer, styrene-ethylene-butylene-styrene block copolymer, styrene-ethylene-propylene-styrene block copolymer, and amorphous polypropylene with a tackifier, a plasticizer, an antioxidant, or the like.

Examples of the adhesive emulsion include a polymerization product of at least one or more monomers selected from the group consisting of methyl methacrylate, styrene, acrylonitrile, 2-ethylhexyl acrylate, butyl acrylate, butadiene, ethylene, and vinyl acetate. These adhesive binders may be used alone, or may be used in combination of two or more.

The absorber of the present embodiment may further contain an additive such as an inorganic powder (for example, amorphous silica), a deodorant, a dye, a pigment, an antibacterial agent, a fragrance, and a sticking agent. These additives can impart various functions to the absorber. In a case where the water-absorbent resin particles contain inorganic particles, the absorber may contain an inorganic powder in addition to the inorganic particles of the water-absorbent resin particles. Examples of the inorganic powder include silicon dioxide, zeolite, kaolin, and clay.

The shape of the absorber of the present embodiment is not particularly limited, and may be a sheet shape, for example. The thickness of the absorber (for example, thickness of the sheet shaped absorber) may be 0.1 to 20 mm or 0.3 to 15 mm, for example.

In addition to the absorber, the absorbent article of the present embodiment may include a core wrap, a liquid permeable top sheet, and a liquid impermeable back sheet, for example. The core wrap retains the absorber. The liquid permeable top sheet is disposed on the outermost part at the side where the liquid to be absorbed enters. The liquid impermeable back sheet is disposed on the outermost part at the opposite side to the side where the liquid to be absorbed enters.

Examples of the absorbent article include diapers (for example, paper diapers), toilet training pants, incontinence pads, sanitary products (sanitary napkins, tampons, and the like), sweat pads, pet sheets, portal toilet members, and animal excrement treatment materials.

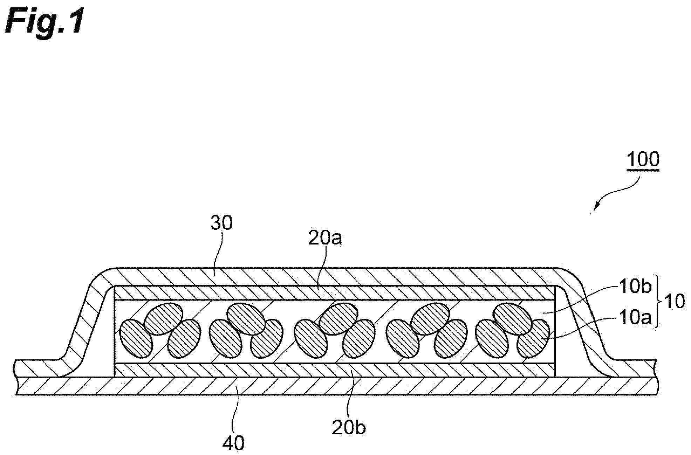

Fig. 1 is a cross-sectional view showing an example of an absorbent article. An absorbent article 100 shown in Fig. 1 includes an absorber 10, core wraps 20a and 20b, a liquid permeable top sheet 30, and a liquid impermeable back sheet 40. In the absorbent article 100, the liquid impermeable back sheet 40, the core wrap 20b, the absorber 10, the core wrap 20a, and the liquid permeable top sheet 30 are laminated in this order. In Fig. 1, there is a portion shown so that there is a gap between the members, but the members may be in close contact with each other without the gap.

The absorber 10 has a water-absorbent resin particle 10a and a fiber layer 10b containing a fibrous substance. The water-absorbent resin particles 10a are dispersed in the fiber layer 10b.

The core wrap 20a is disposed on one surface side of the absorber 10 (an upper side of the absorber 10 in Fig. 1) in a state of being in contact with the absorber 10. The core wrap 20b is disposed on the other surface side of the absorber 10 (a lower side of the absorber 10 in Fig. 1) in a state of being in contact with the absorber 10. The absorber 10 is disposed between the core wrap 20a and the core wrap 20b.

The core wrap 20a and the core wrap 20b each have a main surface having the same size as that of the absorber 10, for example. By using the core wrap, it is possible to maintain shape retainability of the absorber and prevent falloff or flow of the water-absorbent resin particles and the like constituting the absorber. Examples of the core wrap include non-woven fabrics, woven fabrics, tissues, synthetic resin films having liquid permeation holes, and net-like sheets having a mesh, and from the viewpoint of economic efficiency, tissues obtained by wet-type molding pulverized pulp are preferably used.

The liquid permeable top sheet 30 is disposed on the outermost part at the side where the liquid to be absorbed enters. The liquid permeable top sheet 30 is disposed on the core wrap 20a in a state of being in contact with the core wrap 20a. The liquid impermeable back sheet 40 is disposed on the outermost part at the opposite side to the liquid permeable top sheet 30 in the absorbent article 100. The liquid impermeable back sheet 40 is disposed on a lower side of the core wrap 20b in a state of being in contact with the core wrap 20b. The liquid permeable top sheet 30 and the liquid impermeable back sheet 40 have a main surface wider than the main surface of the absorber 10, and outer edges of the liquid permeable top sheet 30 and the liquid impermeable back sheet 40 are present around the absorber 10 and the core wraps 20a and 20b.

Examples of the liquid permeable top sheet 30 include a non-woven fabric and a porous sheet. Examples of the non-woven fabric include thermal bonded non-woven fabrics, air through non-woven fabrics, resin bonded non-woven fabrics, spunbond non-woven fabrics, melt-blown non-woven fabrics, spunbond/melt-blown/spunbond non-woven fabrics, airlaid non-woven fabrics, spunlace non-woven fabrics, and point-bonded non-woven fabrics. Among these, thermal bonded non-woven fabrics, air through non-woven fabrics, spunbond non-woven fabrics, and spunbond/melt-blown/spunbond non-woven fabrics are preferably used.

As a constituent material for the liquid permeable top sheet 30, resins or fibers known in the technical field can be used, and from the viewpoint of liquid permeability, flexibility, and strength when used in the absorbent article, examples include polyolefin such as polyethylene (PE) and polypropylene (PP); polyester such as polyethylene terephthalate (PET), polytrimethylene terephthalate (PTT), and polyethylene naphthalate (PEN); polyamide such as nylon; rayon; other synthetic resins or synthetic fibers; and fibers such as cotton, silk, hemp, and pulp (cellulose). As the constituent material, from the viewpoint of increasing strength of the liquid permeable top sheet 30, synthetic fibers are preferably used, and among these, polyolefin or polyester is preferable. These materials may be used alone, or two or more materials may be used in combination.

It is desirable that a non-woven fabric used for the liquid permeable top sheet 30 have appropriate hydrophilicity from the viewpoint of improving liquid absorption performances of the absorbent article. From this viewpoint, one having the hydrophilicity of 5 to 200 is preferable, and one having the hydrophilicity of 10 to 150 is more preferable, where the hydrophilicity is measured according to "Hydrophilicity of Non-Woven Fabric" (in accordance with Pulp and Paper Test Method No. 68 (2000)) disclosed in PCT International Publication No. WO2011/086843. Among the above-mentioned non-woven fabrics, a non-woven fabric having such a hydrophilicity may be formed using one, such as rayon fibers, which shows an appropriate hydrophilicity by itself; or may be formed using one obtained by hydrophilizing hydrophobic chemical fibers such as polyolefin fibers and polyester fibers by a known method to impart an appropriate hydrophilicity.

Examples of the method of hydrophilizing chemical fibers include a method of obtaining a non-woven fabric by a spunbond technique using one obtained by mixing a hydrophilizing agent to hydrophobic chemical fibers in a spunbond non-woven fabric, a method of using a hydrophilizing agent when producing a spunbond non-woven fabric with hydrophobic chemical fibers, and a method of obtaining a spunbond non-woven fabric with hydrophobic chemical fibers, and thereafter impregnating with a hydrophilizing agent. As the hydrophilizing agent, anionic surfactants such as aliphatic sulfonic acid salts and higher alcohol sulfuric acid ester salts; cationic surfactants such as quaternary ammonium salts; nonionic surfactants such as polyethylene glycol fatty acid esters, polyglycerin fatty acid esters, and sorbitan fatty acid esters; silicone surfactants such as polyoxyalkylene-modified silicone; stain release agents formed of polyester-based, polyamide-based, acrylic-based, or urethane-based resin; and the like are used.

It is preferable that a non-woven fabric used for the liquid permeable top sheet 30 is moderately bulky and has a large fabric weight per unit area from the viewpoint of imparting favorable liquid permeability, flexibility, strength, and cushioning properties to the absorbent article, and accelerating a liquid permeation rate of the absorbent article. The fabric weight per unit area of the non-woven fabric is preferably 5 to 200 $g/m^2$, more preferably 8 to 150 $g/m^2$, and further more preferably 10 to 100 $g/m^2$. Furthermore, the thickness of the non-woven fabric is preferably 20 to 1400 $\mu$m, more preferably 50 to 1200 $\mu$m, and further more preferably 80 to 1000 $\mu$m.

The liquid impermeable back sheet 40 prevents a liquid absorbed by the absorber 10 from leaking to the outside from the back sheet 40 side. For the liquid impermeable back sheet 40, it is possible to use liquid impermeable films mainly composed of polyolefin resins such as polyethylene (PE) and polypropylene (PP); breathable resin films; composite films in which a breathable resin film is bonded to a non-woven fabric such as a spunbond non-woven fabric and a spunlace non-woven fabric; spunbond/melt-blown/spunbond (SMS) non-woven fabrics in which a water-resistant melt blown non-woven fabric is sandwiched between high-strength spunbond non-woven fabrics; and the like. For the back sheet 40, it is possible to use a resin film having the fabric weight per unit area of 10 to 50 $g/m^2$ and mainly made of low-density polyethylene (LDPE) resin from the viewpoint of ensuring flexibility so as not to impair a sense of wearing the absorbent article. Furthermore, in a case where a breathable material is used, dampness generated at the time of wearing is reduced, and thereby discomfort to a wearer can also be reduced.

The magnitude relationship between the absorber 10, the core wraps 20a and 20b, the liquid permeable top sheet 30, and the liquid impermeable back sheet 40 is not particularly limited, and is appropriately adjusted according to the use of the absorbent article or the like. In addition, the method of retaining the shape of the absorber 10 using the core wraps 20a and 20b is not particularly limited, and as shown in Fig. 1, the absorber may be sandwiched by a plurality of core wraps, and the absorber may be covered by one core wrap.

The absorber 10 may be adhered to the liquid permeable top sheet 30. By adhering the absorber 10 and the liquid permeable top sheet 30, a liquid is more smoothly guided to the absorber, and thereby the absorbent article further better in preventing liquid leakage is easily obtained. In a case where the absorber 10 is sandwiched or covered by the core wrap, it is preferable that at least the core wrap and the liquid permeable top sheet 30 are adhered to each other, and it is more preferable that the core wrap and the absorber 10 are further adhered to each other. Examples of a method of adhering include a method of adhering by applying a hot melt adhesive to the liquid permeable top sheet 30 in a width direction thereof at predetermined intervals in a shape such as a longitudinal direction striped shape and a spiral shape; and a method of adhering using a water-soluble binder selected from starch, carboxymethyl cellulose, polyvinyl alcohol, polyvinylpyrrolidone, and other water-soluble polymers. In addition, in a case where the absorber 10 contains thermal bonding synthetic fibers, a method of adhering by thermal bonding thereof may be adopted.

A method for producing water-absorbent resin particles of the present embodiment may include sorting out water-absorbent resin particles based on the absorption efficiency measured by the above-mentioned method. Specifically, sorting may be, for example, sorting out water-absorbent resin particles in which the absorption efficiency index is 0 to 15. The above-mentioned production method may include a step of measuring the absorption efficiency index of the water-absorbent resin particles. As properties of the water-absorbent resin particles to be sorted out, those satisfying the above-described aspects of the water-absorbent resin particles (for example, a 5-minute value of non-pressurization DW in a specific range, a physiological saline absorption amount in a specific range, a water absorption amount under load in a specific range, and the like) may be sorted out.

One aspect of the present embodiment can also be said to be a method for improving water absorption performance of an absorbent article containing water-absorbent resin particles in an inclined state, the method including adjusting an absorption efficiency index of the water-absorbent resin particles measured by the above-described measurement method. Specific examples of the improvement of water absorption performance of the absorbent article in the inclined state specifically include improvement of a water absorption rate and improvement of an absorption amount of the absorbent article in the inclined state. More specific measurement method of items required for calculating the absorption efficiency index will be described in Examples to be described later. The method for improving water absorption performance of the absorbent article in the inclined state may further include, for example, adjusting the absorption efficiency of the water-absorbent resin particles to the range of 0 to 15, adjusting the 5-minute value of non-pressurization DW to 30 ml/g or more, and adjusting the physiological saline absorption amount to 30 g/g or more. A specific example of the method for producing the water-absorbent resin particles having these predetermined properties is as described above. Adjusting the absorption efficiency index of the water-absorbent resin particles to the range of 1 to 15 can be performed by, for example, selecting production conditions for the water-absorbent resin particles so that each of the particles of water-absorbent resin is uniformly crosslinked, and the uniformity of crosslinking in the particles is further improved.

According to the present embodiment, it is possible to provide a method for producing an absorber by using the water-absorbent resin particles obtained by the above-mentioned method for producing water-absorbent resin particles. The method for producing an absorber of the present embodiment includes a particle producing step of obtaining water-absorbent resin particles by the above-mentioned method for producing water-absorbent resin particles. The method for producing an absorber of the present embodiment may include a step of mixing the water-absorbent resin particles and a fibrous substance after the particle producing step. According to the present embodiment, it is possible to provide a method for producing an absorbent article by using the absorber obtained by the above-mentioned method for producing an absorber. The method for producing an absorbent article of the present embodiment includes an absorber producing step of obtaining an absorber by the above-mentioned method for producing an absorber. The method for producing an absorbent article of the present embodiment may include a step of obtaining an absorbent article by using the absorber and other constituent member for an absorbent article after the absorber producing step, and in this step, for example, an absorbent article is obtained by laminating the absorber and other constituent member for an absorbent article with each other.

## Examples

Hereinafter, contents of the present invention will be described in further detail using examples and comparative examples, but the present invention is not limited to the following examples.

[Example 1]

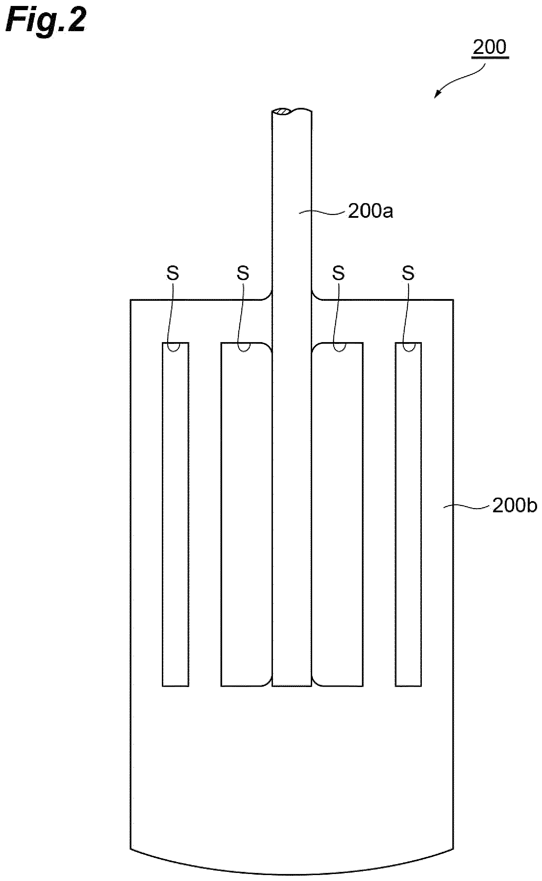

A round-bottomed cylindrical separable flask with the inner diameter of 11 cm and the internal volume of 2 L equipped with a reflux cooling device, a dropping funnel, a nitrogen gas introduction tube, and a stirrer was prepared. The stirrer was equipped with a stirring blade 200 of which an outline is shown in Fig. 2. The stirring blade 200 includes a shaft 200a and a flat plate portion 200b. The flat plate portion 200b is welded to the shaft 200a and has a curved distal end. Four slits S extending along an axial direction of the shaft 200a are formed in the flat plate portion 200b. The four slits S are arranged in a width direction of the flat plate portion 200b, where the width of the two slits S at the inner side is 1 cm, and the width of the two slits S at the outer side is 0.5 cm. The length of the flat plate portion 200b is about 10 cm, and the width of the flat plate portion 200b is about 6 cm. In the prepared separable flask, 293 g of n-heptane, and 0.736 g of a maleic anhydride-modified ethylene-propylene copolymer (manufactured by Mitsui Chemicals, Inc., Hi-Wax 1105A) as a dispersant were mixed. The dispersant was dissolved in n-heptane by raising the temperature to 80°C while stirring the mixture in the separable flask with the stirrer. The formed solution was cooled to 50°C.

Meanwhile, 92.0 g (1.03 mol) of an aqueous solution of 80.5% by mass acrylic acid was put into a beaker having the internal volume of 300 mL as a water-soluble ethylenically unsaturated monomer, and while cooling from the outside, 147.7 g of an aqueous solution of 20.9% by mass sodium hydroxide was added dropwise into the beaker to perform 75 mol% of neutralization. Thereafter, 0.092 g of hydroxyethyl cellulose (Sumitomo Seika Chemicals Co., Ltd., HEC AW-15F) as a thickener, 0.0648 g (0.272 mmol) of sodium persulfate as a water-soluble radical polymerization agent, and 0.0156 g (0.090 mmol) of ethylene glycol diglycidyl ether as an internal crosslinking agent were added and dissolved to prepare a first stage aqueous liquid.

The first stage aqueous liquid was added into the above-mentioned separable flask, and stirring was performed for 10 minutes. Thereafter, 0.736 g of sucrose stearic acid ester (Mitsubishi-Chemical Foods Corporation, Ryoto Sugar Ester S-370, HLB: 3) as a surfactant was heat-dissolved in 6.62 g of n-heptane to obtain a surfactant solution. The above-mentioned surfactant solution was added into the above-mentioned separable flask, and the inside of the system was sufficiently replaced with nitrogen while stirring at the rotation speed of 350 rpm of the stirrer. Thereafter, the above-mentioned separable flask was immersed in a water bath at 70°C to raise the temperature, and polymerization was performed for 60 minutes to obtain a first stage polymerization slurry solution.

128.8 g (1.44 mol) of an aqueous solution of 80.5% by mass acrylic acid was put into another beaker having the internal volume of 500 mL as a water-soluble ethylenically unsaturated monomer, and while cooling from the outside, 159.0 g of an aqueous solution of 27% by mass sodium hydroxide was added dropwise to perform 75 mol% of neutralization. In the beaker after the neutralization, 0.0907 g (0.381 mmol) of sodium persulfate as a water-soluble radical polymerization initiator and 0.0129 g (0.074 mmol) of ethylene glycol diglycidyl ether as an internal crosslinking agent were added and dissolved to prepare a second stage aqueous liquid.

While stirring at the rotation speed of 650 rpm of the stirrer, the inside of the above-mentioned separable flask system was cooled to 25°C. After cooling, the total amount of the above-mentioned second stage aqueous liquid was added to the first stage polymerization slurry solution, and the inside of the separable flask system was replaced with nitrogen for 30 minutes. Thereafter, the separable flask was immersed again in a water bath at 70°C to raise the temperature, and the polymerization reaction was performed for 60 minutes to obtain a hydrogel-like polymer.

To the hydrogel-like polymer after the second stage polymerization, 0.589 g of an aqueous solution of 45% by mass diethylenetriamine pentaacetic acid pentasodium was added under stirring. Thereafter, the above-mentioned separable flask was immersed in an oil bath set at 125°C, and 254.5 g of water was extracted to the outside of the system while refluxing n-heptane by azeotropic distillation of n-heptane and water. Thereafter, 4.42 g (0.507 mmol) of an aqueous solution of 2% by mass ethylene glycol diglycidyl ether was added into the above-mentioned separable flask as a surface crosslinking agent, and the mixture was maintained at 83°C for 2 hours.

Thereafter, drying was performed by evaporating n-heptane at 125°C to obtain polymer particles (dried product). These polymer particles were passed through a sieve having the opening of 850 $\mu$m, 0.5% by mass of amorphous silica (Oriental Silicas Corporation, Tokusil NP-S) with respect to the mass of the polymer particles was mixed with the polymer particles to obtain 231.8 g of water-absorbent resin particles containing amorphous silica. The median particle diameter of the water-absorbent resin particles was 368 $\mu$m, the water absorption amount under the load of 4.14 kPa was 25 ml/g, the water absorption amount under the load of 2.07 kPa was 33 ml/g, and the 5-minute value of non-pressurization DW was 55 ml/g.

[Example 2]

231.2 g of water-absorbent resin particles were obtained in the same manner as that in Example 1, except that, in preparation of a first stage aqueous liquid, the use amount of ethylene glycol diglycidyl ether as an internal crosslinking agent was changed to 0.010 g (0.057 mmol); in preparation of a second stage aqueous liquid, the use amount of ethylene glycol diglycidyl ether as an internal crosslinking agent was changed to 0.0116 g (0.067 mmol); the amount of water extracted to the outside of the system by azeotropic distillation was changed to 257.2 g; and the amount of amorphous silica mixed with the polymer particles was changed to 0.2% by mass with respect to the mass of the polymer particles. The median particle diameter of the water-absorbent resin particles was 359 $\mu$m, the water absorption amount under the load of 4.14 kPa was 23 ml/g, the water absorption amount under the load of 2.07 kPa was 34 ml/g, and the 5-minute value of non-pressurization DW was 49 ml/g.

[Example 3]

229.0 g of water-absorbent resin particles were obtained in the same manner as that in Example 2, except that, in preparation of a first stage polymerization slurry solution, the rotation speed of the stirrer at the time of nitrogen replacement was changed to 425 rpm; and the amount of water extracted to the outside of the system by azeotropic distillation was changed to 271.0 g. The median particle diameter of the water-absorbent resin particles was 360 µm, the water absorption amount under the load of 4.14 kPa was 11 ml/g, the water absorption amount under the load of 2.07 kPa was 19 ml/g, and the 5-minute value of non-pressurization DW was 45 ml/g.

[Example 4]

230.8 g of water-absorbent resin particles were obtained in the same manner as that in Example 1, except that, in preparation of a first stage aqueous liquid, the use amount of ethylene glycol diglycidyl ether as an internal crosslinking agent was changed to 0.010 g (0.057 mmol); in preparation of a first stage polymerization slurry solution, the rotation speed of the stirrer at the time of nitrogen replacement was changed to 425 rpm; in preparation of a second stage aqueous liquid, a radical polymerization initiator used was changed to 0.0907 g (0.381 mmol) of sodium persulfate, and the use amount of ethylene glycol diglycidyl ether as an internal crosslinking agent was changed to 0.0116 g (0.067 mmol); and the amount of water extracted to the outside of the system by azeotropic distillation was changed to 263.7 g. The median particle diameter of the water-absorbent resin particles was 350 µm, the water absorption amount under the load of 4.14 kPa was 18 ml/g, the water absorption amount under the load of 2.07 kPa was 34 ml/g, and the 5-minute value of non-pressurization DW was 62 ml/g.

[Comparative Example 1]

232.5 g of water-absorbent resin particles was obtained in the same manner as in Example 1 except that, a stirring blade was changed to one having two stages of four inclined paddle blades with the blade diameter of 5 cm; in preparation of a first stage aqueous liquid, a radical polymerization initiator used was changed to 0.092 g (0.339 mmol) of 2,2'-azobis(2-amidinopropane) dihydrochloride and 0.018 g (0.068 mmol) of potassium persulfate, and the addition amount of ethylene glycol diglycidyl ether as an internal crosslinking agent was changed to 0.0046 g (0.026 mmol); in preparation of a first stage polymerization slurry solution, the rotation speed of the stirrer at the time of nitrogen replacement was changed to 550 rpm; in preparation of a second stage aqueous liquid, a radical polymerization initiator used was changed to 0.129 g (0.475 mmol) of 2,2'-azobis(2-amidinopropane) dihydrochloride and 0.026 g (0.095 mmol) of potassium persulfate; after the preparation of the second stage aqueous liquid, the rotation speed of the stirrer at the time of cooling the inside of the separable flask system to 25°C was changed to 1000 rpm; the amount of water extracted to the outside of the system by azeotropic distillation was changed to 207.9 g; and the amount of amorphous silica mixed with the polymer particles was changed to 0.2% by mass with respect to the mass of the polymer particles. The median particle diameter of the water-absorbent resin particles was 360 µm, the water absorption amount under the load of 4.14 kPa was 26 ml/g, the water absorption amount under the load of 2.07 kPa was 30 ml/g, and the 5-minute value of non-pressurization DW was 38 ml/g.

[Comparative Example 2]

229.0 g of water-absorbent resin particles was obtained in the same manner as in Comparative Example 1 except that an amount of water extracted out of the system by azeotropic distillation was changed to 216.7 g. The median particle diameter of the water-absorbent resin particles was 348 µm, the water absorption amount under the load of 4.14 kPa was 27 ml/g, the water absorption amount under the load of 2.07 kPa was 32 ml/g, and the 5-minute value of non-pressurization DW was 47 ml/g.

[Comparative Example 3]

228.5 g of water-absorbent resin particles was obtained in the same manner as in Comparative Example 1 except that an amount of water extracted out of the system by azeotropic distillation was changed to 232.0 g. The median particle diameter of the water-absorbent resin particles was 354 µm, the water absorption amount under the load of 4.14 kPa was 18 ml/g, the water absorption amount under the load of 2.07 kPa was 38 ml/g, and the 5-minute value of non-pressurization DW was 32 ml/g.

The obtained water-absorbent resin particles were measured for the absorption efficiency index, median particle diameter, 5-minute value of non-pressurization DW, water absorption amount under load (4.14 kPa and 2.07 kPa), and liquid accumulation absorption time by the following method. The physiological saline used in the present example is an aqueous solution of 0.9% by mass NaCl.

<Measurement of absorption efficiency index>

[Measurement of physiological saline absorption amount (A)]

The measurement was performed in the environment of the temperature of 25°C $\pm$ 2°C and the humidity of 50% $\pm$ 10%. That is, 500 g of physiological saline was weighed in a beaker having the capacity of 500 mL, and while stirring at 600 r/min using a magnetic stirrer bar (8 mm$\varphi$ $\times$ 30 mm, without a ring), 2.0 g of water-absorbent resin particles was dispersed such that lumps were not generated. By leaving to stand for 60 minutes in a stirred state, the water-absorbent resin particles were sufficiently swollen to obtain a gel. Thereafter, the content of the above-mentioned beaker was filtered using a standard sieve with the opening of 75 $\mu$m of which the mass Wa (g) was previously measured, and the sieve was left to stand for 30 minutes in an inclined state with an inclination angle of about 30 degrees with respect to the horizontal to filter excess water from the gel. The mass Wb (g) of the sieve to which the gel was put was measured, and the physiological saline absorption amount A was obtained by the following formula.

$$\text{Physiological saline absorption amount A [g/g]} = (Wb - Wa)$$
$$\text{[g]/mass of water-absorbent resin particles [g]}$$

[Measurement of water absorption amount (B1) of absorbent article for B1 measurement]

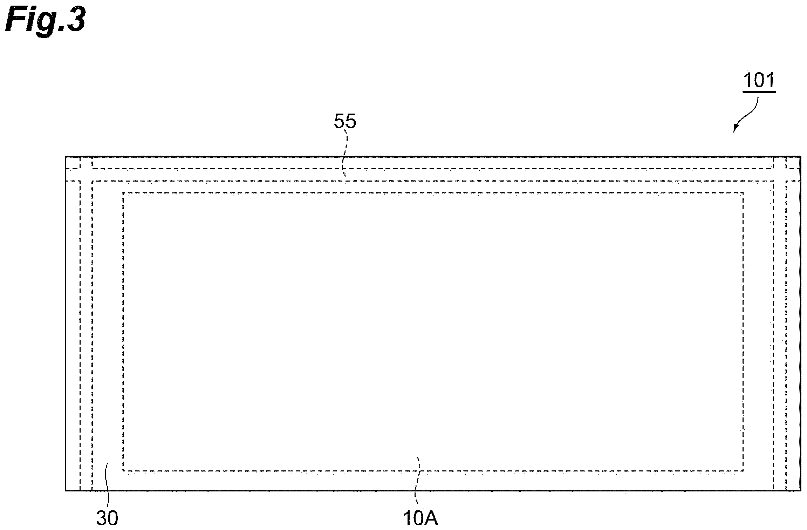

10 g of the water-absorbent resin particles and 8.0 g of pulverized pulp were uniformly mixed by air papermaking using an air flow type mixer (Padformer manufactured by O-tec Co., Ltd.), and thereby a sheet shaped absorber having the size of 40 cm $\times$ 12 cm was produced. The absorber was sandwiched from its upper and lower sides with two sheets of tissue paper (core wrap) having the same size as that of the produced absorber and having the basis weight of 16 g/m$^2$, and the load of 196 kPa was applied to the entire absorber for 30 seconds to obtain an absorber 10A with core wraps. A spunbond-melt-blown-melt-blown-spunbond (SMMS) non-woven fabric (weighing capacity 13 g/m$^2$) having the size of 32.5 cm $\times$ 45.0 cm was prepared as the liquid permeable top sheet 30, and as shown in Fig. 3, this was folded in half while sandwiching the absorber 10A with core wraps therebetween. An end 55 along the three open sides, other than the crease, of the liquid permeable top sheet 30 folded in half was crimped by a heat sealer to obtain an absorbent article 101 for B1 measurement containing the water-absorbent resin particles and constituted of the absorber 10A with core wraps and the liquid permeable top sheet 30 wrapping the absorber 10A with core wraps.

According to the above-mentioned method for measuring the physiological saline absorption amount (A), when measurement was performed using 2.0 g of pulverized pulp used for producing the absorbent articles for B1 measurement and B2 measurement instead of the water-absorbent resin particles, the physiological saline absorption amount of the pulverized pulp was 27 g/g.

A wire mesh (opening size: 20 mm $\times$ 20 mm, wire diameter 3 mm) and 20 L of physiological saline were put in a bat, and the liquid temperature was adjusted to 25.0°C $\pm$ 0.2°C. Next, the absorbent article for B1 measurement was widely disposed on the wire mesh and immersed in the aqueous solution of physiological saline for 30 minutes. Thereafter, the wire mesh was lifted together with the absorbent article, draining was performed for 5 minutes, and then the mass of the absorbent article after water absorption was measured. The water absorption amount [g] of the absorbent article is a difference in masses of the absorbent article before and after water absorption, and is calculated by the following formula.

Water absorption amount (B1) of absorbent article for B1 measurement = mass of absorbent article after water absorption (g) - mass of absorbent article before water absorption (g)

[Measurement of water absorption amount (B2) of absorbent article not containing water-absorbent resin particles]

An absorbent article for B2 measurement not containing the water-absorbent resin particles was obtained in the same manner as in the above-mentioned B1 except that 10 g of the water-absorbent resin particles was not contained. In the same manner as in the above-mentioned B1, the mass of the absorbent article before and after water absorption was measured, and the water absorption amount (B2) of the absorbent article not containing the water-absorbent resin particles was calculated. The results are shown in Table 1.

From the above-mentioned B1 and B2, the apparent water absorption amount (C) per 1 g of the water-absorbent resin particles was calculated by the following formula.

$$C\ [g/g] = (B1 - B2)/10$$

From A and C obtained above, an absorption efficiency index was further obtained by the following formula. The results are shown in Table 1.

$$\text{Absorption efficiency index} = [(C - A)/A] \times 100$$

<Measurement of median particle diameter>

In the environment of the temperature of 25°C ± 2°C and the humidity of 50% ± 10%, the above-mentioned median particle diameter of the water-absorbent resin particles was measured by the following procedure. That is, JIS standard sieves were combined in the following order from the top: a sieve having the opening of 600 $\mu$m, a sieve having the opening of 500 $\mu$m, a sieve having the opening of 425 $\mu$m, a sieve having the opening of 300 $\mu$m, a sieve having the opening of 250 $\mu$m, a sieve having the opening of 180 $\mu$m, a sieve having the opening of 150 $\mu$m, and a tray. 50 g of the water-absorbent resin particles was put in the topmost sieve among the combined sieves, and classification was performed using a Ro-tap shaker (manufactured by Iida-seisakusho Japan Corporation) according to JIS Z 8815 (1994). After the classification, the mass of the particles remaining on each of the sieves was calculated as a mass percentage with respect to the total amount to determine a particle size distribution. The relationship between the opening of the sieve and the integrated value of the mass percentage of the particles remaining on the sieve was plotted on a logarithmic probability paper by integrating in the order from the one having the largest particle diameter on the sieve with respect to this particle size distribution. By connecting the plots on the probability paper with a straight line, the particle diameter corresponding to the cumulative mass percentage of 50% by mass was obtained as the median particle diameter.

<Measurement of non-pressurization DW>

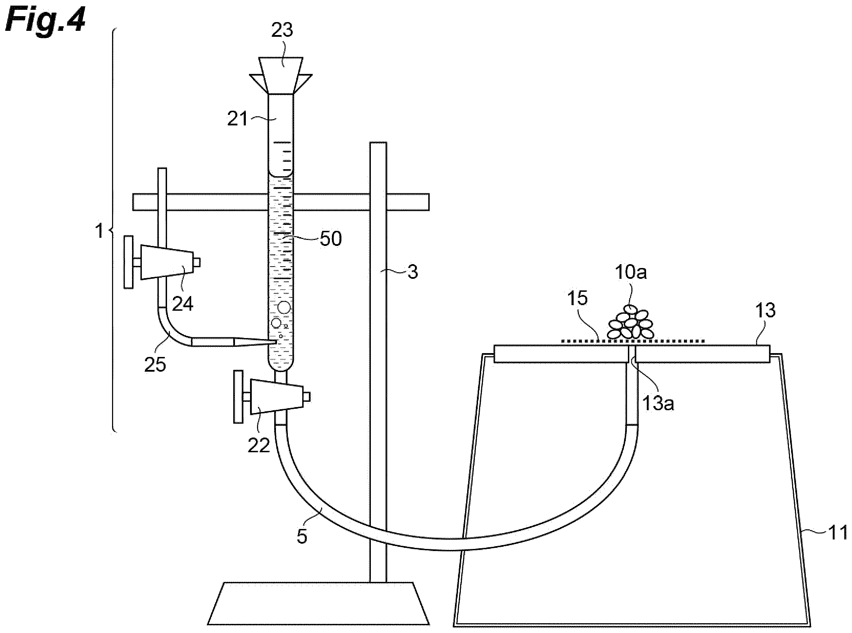

The non-pressurization DW of the particles of water-absorbent resin was measured using a measurement device shown in Fig. 4. The measurement was performed five times for one type of water-absorbent resin particles, and an average value of three measurement values excluding a minimum value and a maximum value was obtained.

The measurement device has a burette unit 1, a conduit 5, a measurement table 13, a nylon mesh sheet 15, a stand 11, and a clamp 3. The burette unit 1 has a burette tube 21 on which a scale is engraved, a rubber stopper 23 for sealing the opening at the upper part of the burette tube 21, a cock 22 connected to the distal end of the lower part of the burette tube 21, an air introduction tube 25 connected to the lower part of the burette tube 21, and a cock 24. The burette unit 1 is fixed by the clamp 3. The flat plate-shaped measurement table 13 has a through hole 13a having the diameter of 2 mm and formed in the central portion thereof, and is supported by the height-variable stand 11. The through hole 13a of the measurement table 13, and the cock 22 of the burette unit 1 are connected by the conduit 5. The inner diameter of the conduit 5 is 6 mm.

The measurement was performed in the environment of the temperature of 25°C ± 2°C and the humidity of 50% ± 10%. First, the cock 22 and the cock 24 of the burette unit 1 were closed, and physiological saline (0.9% by mass saline) 50 adjusted to 25°C was put into the burette tube 21 from the opening at the upper part of the burette tube 21. The concentration 0.9% by mass of the saline is a concentration based on the mass of the saline. The opening of the burette tube 21 was sealed with the rubber stopper 23, and then the cock 22 and the cock 24 were opened. The inside of the conduit 5 was filled with the physiological saline 50 to prevent air bubbles from entering. The height of the measurement table 13 was adjusted so that the height of the water surface of the physiological saline reached the inside of the through hole 13a was the same as the height of the upper surface of the measurement table 13. After the adjustment, the height of the water surface of the physiological saline 50 in the burette tube 21 was read by the scale on the burette tube 21, and this position was defined as a zero point (value read at 0 seconds).

The nylon mesh sheet 15 (100 mm × 100 mm, 250 mesh, thickness about 50 $\mu$m) was laid in the vicinity of the through hole 13a on the measurement table 13, and a cylinder having the inner diameter of 30 mm and the height of 20 mm was placed on the central portion thereof. 1.00 g of water-absorbent resin particles 10a were uniformly scattered in this cylinder. Thereafter, the cylinder was carefully removed to obtain a sample in which the water-absorbent resin particles 10a were dispersed in a circle shape in the central portion of the nylon mesh sheet 15. Then, the nylon mesh sheet 15 on which the water-absorbent resin particles 10a were placed was moved at a high speed to the extent that the water-absorbent resin particles 10a did not dissipate so that the center of the nylon mesh sheet was at the position of the through hole 13a, and the measurement was started. The timing when air bubbles were first introduced from the air introduction tube 25 into the burette tube 21 was defined as the start of water absorption (0 seconds).

The amount of reduction in the physiological saline 50 in the burette tube 21 (that is, the amount of the physiological saline absorbed by the water-absorbent resin particles 10a) was sequentially read by units of 0.1 mL, and the amount of reduction Wc (g) of the physiological saline 50 was read after 5 minutes from the start of water absorption by the water-absorbent resin particles 10a. A 5-minute value of non-pressurization DW was obtained from Wc by the following formula. The non-pressurization DW is a water absorption amount per 1.00 g of the water-absorbent resin particles 10a.

$$\text{5-minute value of non-pressurization DW (mL/g)} = \text{Wc}/1.00$$

<Measurement of water absorption amount under load of 4.14 kPa or load of 2.07 kPa>

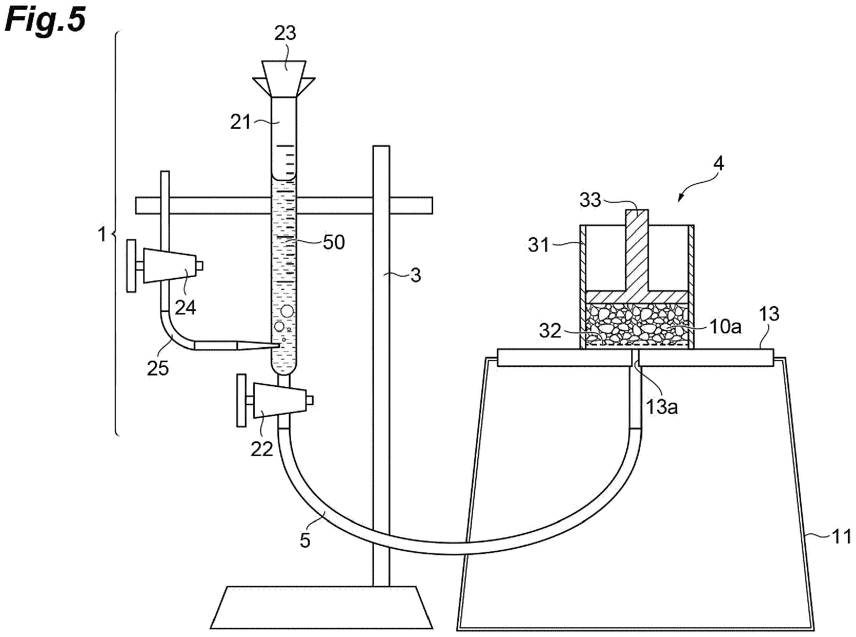

The physiological saline absorption amount under the load of 4.14 kPa or load of 2.07 kPa (water absorption amount under load) was measured using a measurement device schematically shown in Fig. 5. The measurement was performed twice for one type of water-absorbent resin particles, and an average value was obtained. The measurement device includes a burette unit 1, a clamp 3, a conduit 5, a stand 11, a measurement table 13, and a measurement unit 4 placed on the measurement table 13. The burette unit 1 has a burette tube 21 on which a scale is engraved, a rubber stopper 23 for sealing the opening at the upper part of the burette tube 21, a cock 22 connected to the distal end of the lower part of the burette tube 21, an air introduction tube 25 connected to the lower part of the burette tube 21, and a cock 24. The burette unit 1 is fixed by the clamp 3. The flat plate-shaped measurement table 13 has a through hole 13a having the diameter of 2 mm and formed in the central portion thereof, and is supported by the height-variable stand 11. The through hole 13a of the measurement table 13, and the cock 22 of the burette unit 1 are connected by the conduit 5. The inner diameter of the conduit 5 is 6 mm.

The measurement unit 4 has a cylinder 31 made of acrylic resin, a polyamide mesh 32 adhered to one opening of the cylinder 31, and a weight 33 that can move up and down in the cylinder 31. The cylinder 31 is placed on the measurement table 13 with the polyamide mesh 32 therebetween. The inner diameter of the cylinder 31 is 20 mm. The opening of the polyamide mesh 32 is 75 $\mu$m (200 mesh). The weight 33 has the diameter of 19 mm and the mass of 119.6 g or 59.8 g, and can apply the load of 4.14 kPa or load of 2.07 kPa to the water-absorbent resin particles 10a uniformly disposed on the polyamide mesh 32 as described later.

Measurement of the water-absorbing ability of physiological saline under the load of 4.14 kPa or load of 2.07 kPa by the measurement device shown in Fig. 5 was performed in the environment of the temperature of 25°C $\pm$ 2°C and the humidity of 50% $\pm$ 10%. First, the cock 22 and the cock 24 of the burette unit 1 were closed, and physiological saline adjusted to 25°C was put into the burette tube 21 from the opening at the upper part of the burette tube 21. Subsequently, the opening at the upper part of the burette tube 21 was sealed with the rubber stopper 23, and then the cock 22 and the cock 24 were opened. The inside of the conduit 5 was filled with the physiological saline 50 to prevent air bubbles from entering. The height of the measurement table 13 was adjusted so that the height of the water surface of the physiological saline reached the inside of the through hole 13a was the same as the height of the upper surface of the measurement table 13. After the adjustment, the height of the water surface of the physiological saline 50 in the burette tube 21 was read by the scale on the burette tube 21, and this position was defined as a zero point (value read at 0 seconds).

In the measurement unit 4, 0.10 g of the water-absorbent resin particles 10a was uniformly disposed on the polyamide mesh 32 in the cylinder 31, the weight 33 was disposed on the water-absorbent resin particles 10a, and the cylinder 31 was installed such that the central portion thereof coincided with a conduit port at the central portion of the measurement table 13. The amount of reduction Wd (ml) of physiological saline in the burette tube 21 after 2 hours from the start of absorbing the physiological saline by the water-absorbent resin particles 10a from the conduit 5 (that is, the amount of the physiological saline absorbed by the water-absorbent resin particles 10a) was read, and the water-absorbing ability of physiological saline of the water-absorbent resin particles 10a under the load of 4.14 kPa or load of 2.07 kPa was calculated by the following formula.

Water-absorbing ability of physiological saline (ml/g) under load of 4.14 kPa or load of 2.07 kPa = Wd (ml)/mass of water-absorbent resin particles (g)

<Measurement of liquid accumulation absorption time>

[Preparation of artificial urine]

In a solution, which was obtained by blending and dissolving the following components in ion-exchanged water so that inorganic salts were present as shown below, a small amount of Blue No. 1 was further blended to prepare artificial urine (test solution). The following concentrations are concentrations based on the total mass of the artificial urine.

Composition of artificial urine
NaCl: 0.780% by mass
CaCl2: 0.022% by mass
MgSO4: 0.038% by mass
Blue No. 1: 0.002% by mass

[Production of absorbent article for evaluation]

4.2 g of the water-absorbent resin particles and 3.9 g of pulverized pulp were uniformly mixed by air papermaking using an air flow type mixer (Padformer manufactured by O-tec Co., Ltd.), and thereby a sheet shaped absorber having the size of 10 cm $\times$ 15 cm was produced. The above-mentioned absorber was sandwiched with two sheets of tissue paper having the basis weight of 16 g/m$^2$ and having the same size as that of the produced absorber, and a laminate was obtained by applying the load of 588 kPa to the entire absorber for 30 seconds. An air-through type porous liquid permeable sheet, which was made of polyethylene-polypropylene, had the basis weight of 22 g/m$^2$, and had the same size as that of the above-mentioned absorber, was disposed on the laminate. Furthermore, a liquid impermeable sheet made of polyethylene and having the size of 10 cm $\times$ 15 cm was attached to the surface at the opposite side to the liquid permeable sheet of the above-mentioned laminate to obtain an absorbent article for liquid accumulation absorption time measurement.

[Liquid accumulation absorption test]

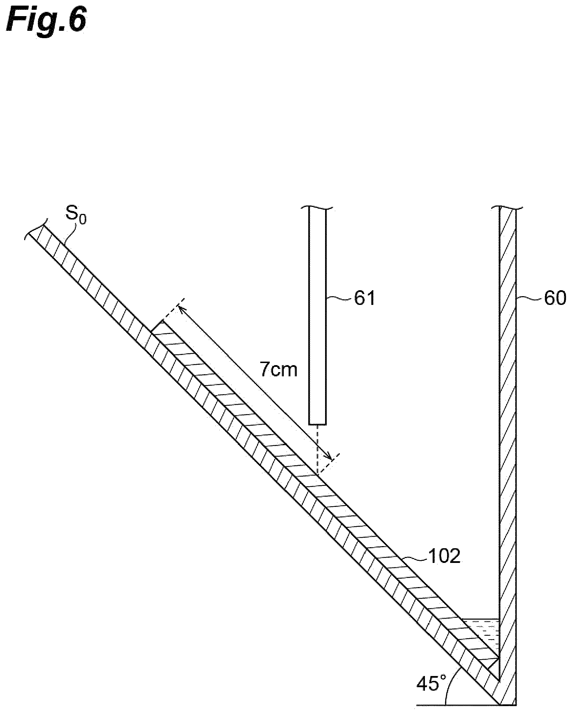

Fig. 6 is a schematic cross-sectional view showing a method for measuring a liquid accumulation absorption time. An instrument for liquid accumulation absorption time measurement shown in Fig. 6 has an inclined plate $S_0$ made of acrylic resin and inclined by 45° with respect to a horizontal plane, and a perpendicular plate (vertical plate) 60 made of acrylic resin and provided vertically. The inclined plate $S_0$ and the perpendicular plate 60 are fixed at the lower end. The depth width of the inclined plate $S_0$ and the perpendicular plate 60 is 11 cm, and both ends are closed with a transparent acrylic plate so that a liquid can be accumulated in the instrument. The present test is to evaluate the absorption ability until artificial urine flows down and the ability to suck up artificial urine accumulated at the bottom in the measurement container without being completely absorbed by injecting the above-mentioned artificial urine by a liquid sending pump from vertically above the absorbent article 102 for evaluation placed on the inclined plate $S_0$ in the acrylic measurement instrument.

The liquid accumulation absorption time was measured by the following method. The measurement was performed in the environment of the temperature of 25°C $\pm$ 2°C and the humidity of 50% $\pm$ 10%. The absorbent article 102 for evaluation was bonded to the inclined plate $S_0$ so that a longitudinal direction was an inclined direction, the liquid permeable sheet surface was the upper side, and the lower end of the absorbent article 102 for evaluation was in contact with the lower end of the instrument. Using a liquid sending pump (manufactured by INTEGRA Biosciences AG., DOSE IT P910, injection slot diameter 0.5 cm$\varphi$) 61, at a flow rate of 4 mL/sec, from the position 1 cm vertically above the injection site, 40.0 ml of artificial urine adjusted to the liquid temperature of 25°C $\pm$ 1°C was injected to the position 7 cm along the absorbent article 102 for evaluation from the center of upper end of the absorbent article 102 for evaluation (first injection). The time (seconds), from the start of the artificial urine injection until the artificial urine accumulated at the bottom in the measurement container was completely absorbed, was measured and used as the liquid accumulation absorption time (seconds) of the absorbent article. The completion of absorption of artificial urine was visually confirmed. The liquid accumulation absorption time is preferably short. In a case where the liquid accumulation absorption time from the start of the artificial urine injection was shorter than 60 seconds, the operation of performing addition of 40.0 ml of artificial urine again in the same manner was repeated after 1 minute from the start of the artificial urine injection, and the number of injections until the liquid accumulation absorption time became 60 seconds or longer was used as the evaluation standard. The surface of the inclined plate $S_0$ was smooth, and a liquid did not stay on or was not absorbed by the inclined plate $S_0$ at the time of the measurement. The results are shown in Table 2.

[Table 1]

| | A | C | B1 | B2 | Absorption efficiency index |
|---|---|---|---|---|---|
| | Physiological saline absorption amount [g/g] | Apparent water absorption amount per 1 g of water-absorbent resin particles [g/g] | Water absorption amount of absorbent article for B1 measurement [g] | Water absorption amount of absorbent article for B2 measurement [g] | |
| Example 1 | 57 | 61 | 885 | | 7 |
| Example 2 | 61 | 64 | 920 | | 5 |
| Example 3 | 69 | 70 | 981 | | 1 |
| Example 4 | 63 | 71 | 989 | | 13 |
| Comparative Example 1 | 51 | 50 | 777 | 277 | -2 |
| Comparative Example 2 | 59 | 57 | 847 | | -3 |
| Comparative Example 3 | 68 | 61 | 889 | | -10 |

[Table 2]

| | Liquid accumulation absorption time (sec) | | | | | |
|---|---|---|---|---|---|---|
| | First injection | Second injection | Third injection | Fourth injection | Fifth injection | Sixth injection |
| Example 1 | 0 | 16 | 17 | 23 | 41 | 61 |
| Example 2 | 0 | 17 | 18 | 25 | 43 | 61 |
| Example 3 | 0 | 19 | 21 | 27 | 46 | 60 |
| Example 4 | 0 | 14 | 18 | 23 | 36 | 63 |
| Comparative Example 1 | 0 | 19 | 31 | 68 | - | - |
| Comparative Example 2 | 0 | 20 | 33 | 68 | - | - |
| Comparative Example 3 | 0 | 22 | 35 | 67 | - | - |

In the absorbent article using the water-absorbent resin particles of the examples in which the absorption efficiency index is in the range of 0 to 15, 6 times of artificial urine injection was possible in the liquid accumulation absorption test, and it was confirmed that the absorbent article has better water absorption performance in the inclined state. On the other hand, in the absorbent article using the water-absorbent resin particles of the comparative examples in which the absorption efficiency index is less than 0, the liquid accumulation absorption time from the start of the fourth artificial urine injection was 60 seconds or longer in the liquid accumulation absorption test.

**Reference Signs List**

1: burette unit,
3: clamp,
4: measurement unit,
5: conduit,

10: absorber,
10A: absorber with core wraps,
10a: water-absorbent resin particle,
10b: fiber layer,
11: stand,
13: measurement table,
13a: through hole,
15: nylon mesh sheet,
20a, 20b: core wrap,
21: burette tube,
22: cock,
23: rubber stopper,
24: cock,
25: air introduction tube,
30: liquid permeable top sheet,
31: cylinder,
32: polyamide mesh,
33: weight,
40: liquid impermeable back sheet,
50: physiological saline,
55: end,
60: perpendicular plate,
61: liquid sending pump,
100: absorbent article,
101: absorbent article for B1 measurement,
102: absorbent article for evaluation,
200: stirring blade,
200a: shaft,
200b: flat plate portion,
S: slit,
So: inclined plate.

**Claims**

1. Water-absorbent resin particles, having an absorption efficiency index of 0 to 15,

   the absorption efficiency index being represented by the following formula,

$$\text{absorption efficiency index} = [(C - A)/A] \times 100,$$

   A: a physiological saline absorption amount of the water-absorbent resin particles,
   C: an apparent water absorption amount per 1 g of the water-absorbent resin particles = (B1 - B2)/10,
   B1: a water absorption amount of an absorbent article for B1 measurement containing the water-absorbent resin particles and including a sheet shaped absorber formed by uniformly mixing 10 g of the water-absorbent resin particles and 8 g of pulverized pulp by air mixing, and
   B2: a water absorption amount of an absorbent article for B2 measurement having the same structure as the absorbent article for B1 measurement except 10 g of the water-absorbent resin particles are not contained.

2. The water-absorbent resin particles according to claim 1, wherein a 5-minute value of non-pressurization DW is 30 ml/g or more.

3. The water-absorbent resin particles according to claim 1 or 2, wherein a physiological saline absorption amount is 30 g/g or more.

4. An absorber comprising the water-absorbent resin particles according to any one of claims 1 to 3.

**5.** An absorbent article comprising the absorber according to claim 4.

**6.** The absorbent article according to claim 5, which is a diaper.

**7.** A method for producing water-absorbent resin particles, the method comprising

sorting out water-absorbent resin particles in which an absorption efficiency index represented by the following formula is 0 to 15,

$$\text{absorption efficiency index} = [(C - A)/A] \times 100,$$

A: a physiological saline absorption amount of the water-absorbent resin particles,
C: an apparent water absorption amount per 1 g of the water-absorbent resin particles = (B1 - B2)/10,
B1: a water absorption amount of an absorbent article for B1 measurement containing the water-absorbent resin particles and including a sheet shaped absorber formed by uniformly mixing 10 g of the water-absorbent resin particles and 8 g of pulverized pulp by air mixing, and
B2: a water absorption amount of an absorbent article for B2 measurement having the same structure as the absorbent article for B1 measurement except 10 g of the water-absorbent resin particles are not contained.

**8.** A method for improving water absorption performance of an absorber containing water-absorbent resin particles in an inclined state, the method comprising

adjusting an absorption efficiency index of the water-absorbent resin particles represented by the following formula to 0 to 15,

$$\text{absorption efficiency index} = [(C - A)/A] \times 100,$$

C: an apparent water absorption amount per 1 g of the water-absorbent resin particles = (B1 - B2)/10,
A: a physiological saline absorption amount of the water-absorbent resin particles,
B1: a water absorption amount of an absorbent article for B1 measurement containing the water-absorbent resin particles and including a sheet shaped absorber formed by uniformly mixing 10 g of the water-absorbent resin particles and 8 g of pulverized pulp by air mixing, and
B2: a water absorption amount of an absorbent article for B2 measurement having the same structure as the absorbent article for B1 measurement except 10 g of the water-absorbent resin particles are not contained.

Fig.1

# *Fig.2*

# Fig.3

55

101

30          10A

EP 3 954 728 A1

**Fig.4**

EP 3 954 728 A1

**Fig.5**

EP 3 954 728 A1

# Fig.6

$S_0$

61

60

7cm

102

45°

## INTERNATIONAL SEARCH REPORT

| International application No. |
|---|
| PCT/JP2020/016741 |

**A. CLASSIFICATION OF SUBJECT MATTER**
Int. Cl. C08J3/16(2006.01)i, A61F13/53(2006.01)i, C08J3/24(2006.01)i, C08L101/14(2006.01)i
FI: C08J3/16, A61F13/53 300, C08J3/24, C08L101/14
According to International Patent Classification (IPC) or to both national classification and IPC

**B. FIELDS SEARCHED**

Minimum documentation searched (classification system followed by classification symbols)
Int. Cl. C08J3/16, A61F13/53, C08J3/24, C08L101/14

Documentation searched other than minimum documentation to the extent that such documents are included in the fields searched
Published examined utility model applications of Japan      1922-1996
Published unexamined utility model applications of Japan    1971-2020
Registered utility model specifications of Japan            1996-2020
Published registered utility model applications of Japan    1994-2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2006/054487 A1 (SUMITOMO SEIKA CHEMICALS CO., LTD.) 26 May 2006, example 1, paragraph [0018] | 1-8 |
| A | WO 2011/086841 A1 (SUMITOMO SEIKA CHEMICALS CO., LTD.) 21 July 2011, production example 1 | 1-8 |
| A | WO 2014/034897 A1 (NIPPON SHOKUBAI CO., LTD.) 06 March 2014, example 1 | 1-8 |
| A | JP 8-509522 A (ALLIED COLLOIDS LTD.) 08 October 1996, example 1 | 1-8 |
| A | JP 2000-26510 A (SANYO CHEMICAL INDUSTRIES, LTD.) 25 January 2000, example 4 | 1-8 |

☒ Further documents are listed in the continuation of Box C.    ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 11.06.2020 | 23.06.2020 |

| Name and mailing address of the ISA/ Japan Patent Office 3-4-3, Kasumigaseki, Chiyoda-ku, Tokyo 100-8915, Japan | Authorized officer Telephone No. |
|---|---|

Form PCT/ISA/210 (second sheet) (January 2015)

**INTERNATIONAL SEARCH REPORT**

| International application No. |
| --- |
| PCT/JP2020/016741 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 60-186506 A (KAO CORP.) 24 September 1985, examples 1-3 | 1-8 |

Form PCT/ISA/210 (continuation of second sheet) (January 2015)

**INTERNATIONAL SEARCH REPORT**
Information on patent family members

International application No.

PCT/JP2020/016741

| Patent Documents referred to in the Report | Publication Date | Patent Family | Publication Date |
|---|---|---|---|
| WO 2006/054487 A1 | 26.05.2006 | US 2008/0119808 A1 example 1, paragraph [0030] EP 1813628 A1 BR PI0517786 A CN 101061145 A | |
| WO 2011/086841 A1 | 21.07.2011 | US 2012/0328862 A1 Production Example 1 EP 2524678 A1 AU 2010342024 A CN 102711697 A KR 10-2012-0115542 A TW 201134505 A DK 2524678 T ES 2539727 T PL 2524678 T | |
| WO 2014/034897 A1 | 06.03.2014 | US 2015/0217270 A1 example 1 EP 2891520 A1 CN 104582833 A KR 10-2015-0048785 A | |
| JP 8-509522 A | 08.10.1996 | US 5684106 A example 1 GB 9322119 D WO 1995/011932 A1 EP 675909 A1 DE 69415651 C AU 7997994 A FI 953086 A BR 9406467 A NO 309937 B AT 175216 E ES 2125493 T ZA 9408464 A AT 175216 T CA 2152362 A SI 675909 T DK 675909 T GR 3029263 T AU 695732 B | |
| JP 2000-26510 A | 25.01.2000 | (Family: none) | |
| JP 60-186506 A | 24.09.1985 | US 4666975 A examples 1-3 GB 8505600 A0 DE 3507775 A FR 2560530 A ES 540973 A CA 1256640 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H5184622 A

- WO 2011086843 A